# EUROPEAN PATENT SPECIFICATION

(11) **EP 2 263 657 B1**
(45) Date of publication and mention of the grant of the patent: **17.07.2013**
(21) Application number: 10163412.9
(22) Date of filing: 20.05.2010
(51) Int. Cl.: A61K 9/20, A61K 9/28, A61K 31/53

(54) **Controlled release lamotrigine formulations**
Lamotriginformulierungen mit gesteuerter Freisetzung
Formulations de lamotrigine à libération contrôlée

(30) Priority: 20.05.2009 IN DE10382009
(43) Date of publication of application: 22.12.2010
(60) Divisional application: 12192095.3
(73) Proprietor: Ranbaxy Laboratories Limited, New Delhi 110019 Delhi (IN)
(72) Inventor: Bhavarisetti, Murali Krishna, 521185, Krishna (IN); Verma, Rajan K., 110043, New Delhi (IN); Singh, Romi Barat, Varanasi, 221002 (IN); Barabde, Umesh Vinayakrao, 444606, Amravati (IN)
(74) Representative: Cronin, Brian Harold John

(56) References cited:
- EP-A1- 2 018 851
- WO-A2-03/104192
- WO-A2-2007/054976
- FR-A1- 2 891 459
- US-A1- 2004 180 088
- US-A1- 2004 192 690

## Description

### Technical Field of the Invention

The present invention relates to controlled release formulations comprising lamotrigine and a process for the preparation thereof.

### Background of the Invention

Lamotrigine is an antiepileptic drug (AED) of the phenyltriazine class. Its chemical name is 3, 5-diamino-6-(2, 3-dichlorophenyl)-1, 2, 4-triazine, which is structurally designated as:

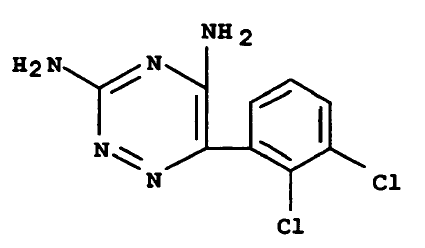

U.S. Patent No. 4,602,017 and European Patent No. 0 021 121 disclose lamotrigine or a pharmaceutically acceptable salt thereof, and also methods of treating convulsions and epilepsy. It also describes process of preparation of lamotrigine. Lamotrigine is indicated for use as adjunctive therapy for partial seizures, the generalized seizures of Lennox-Gastaut syndrome, and primary generalized tonic-clonic seizures in adults and pediatric patients (≥2 years of age). It is also indicated for conversion to monotherapy in adults with partial seizures who are receiving treatment with carbamazepine, phenytoin, phenobarbital, primidone, or valproate as the single AED. Further, it is indicated for the maintenance treatment of Bipolar I Disorder to delay the time to occurrence of mood episodes (depression, mania, hypomania, mixed episodes) in patients treated for acute mood episodes with standard therapy.

Oral immediate release and chewable dispersible tablet formulations of lamotrigine are commercially available in the United States of America under the brand names Lamictal® and Lamictal® CD, respectively, from GlaxoSmithKline, USA. Lamotrigine is rapidly and completely absorbed after oral administration with negligible first-pass metabolism (absolute bioavailability is 98%). The bioavailability is not affected by food. Peak plasma concentrations occur anywhere from 1.4 to 4.8 hours following drug administration. It has been observed and well documented in the art that the existing marketed formulations of lamotrigine (immediate release compositions) lead to severe side-effects, primarily to the rapid increase in blood plasma levels after each dosing. It fact, serious rashes requiring hospitalization and discontinuation of treatment have been reported in association with the use of Lamictal®. To ameliorate the drawbacks associated with conventional immediate release formulations, lamotrigine is formulated into a controlled release once-daily dosage form which is available in the USA, as Lamictal® XR extended release tablets from GlaxoSmithKline Inc.

U.S. Publication No. 2004/0043996 discloses a multiparticulate controlled release formulation, which comprises particles of lamotrigine, a release rate controlling polymer, and a rapidly disintegrating binder, which allows the particles to rapidly disperse in an aqueous environment.

U.S. Publication No. 2004/0192690 describes a sustained release tablet formulation comprising: (i) a matrix core containing lamotrigine and a release retarding swellable and/or gellable polymer, and optionally, (ii) a semi-permeable barrier coating thereby allowing diffusion control of drug release by water insoluble polymer or a partially water soluble polymer, wherein the said outer barrier includes of one or more orifices extending from the outside of the coating substantially completely through said coating but not penetrating said core. Such orifices are generally drilled into the tablet by removing certain portions of the coating; typically the orifices can be produced by mechanical drilling, ultrasonic cutting or laser. The orifices can be any shape, for example oval, round, square or even shaped as text, for example a company logo. Such a formulation is referred as DiffCORE® device. Such devices are also described in U.S. Patent No. 5,004,614.

U.S. Publication No. 2009/0022789 discloses formulations which comprise lamotrigine admixed with a release-equalizing composition comprising a pharmaceutically acceptable organic acid and a release-enhancing polymer, which may be an enteric polymer. Such formulations further contain a release retarding polymer and exhibit a significantly similar rate of release with a similarity factor of at least 50 through the gastrointestinal tract.

In the present case, the inventors have prepared controlled release formulations of lamotrigine using different techniques other than those discussed in the art to formulate a dosage form which manifests fewer side-effects, is patient compliant and can be easily manufactured.

### Summary of the Invention

In one general aspect, the present invention provides for a controlled release formulation, which includes: a) a core comprising of lamotrigine and a hydrophilic rate controlling agent; b) a coating comprising of a pH-dependent polymer and a permeation enhancer; and c) a topcoat comprising of lamotrigine in immediate release form, wherein said coating does not include any orifice.

Embodiments of the present invention may include one or more of the following features. For example, the formulation delivers a therapeutically effective amount of lamotrigine for at least up to 20 hours following a once-daily administration. The formulation is in the form of a unit dosage form.

The present formulation exhibits the following in vitro dissolution profile when measured in a USP type II apparatus with alternate sinkers, at 75 rpm, at a temperature of 37°C±0.5°C in 900mL of 0.1 N hydrochloric acid for the first 2 hours followed by media replacement with pH 6.8 phosphate buffer medium: a) not more than about 15% drug released in 2 hours; b) not more than about 40% drug released in 4 hours; and c) not more than about 60 % drug released in 8 hours.

The hydrophilic rate controlling agent may be alkylcelluloses; hydroxyalkyl alkylcelluloses; carboxyalkylcelluloses; alkali metal salts of carboxyalkylcelluloses; carboxyalkyl alkylcelluloses; carboxyalkylcellulose esters; other natural, semi-synthetic, or synthetic polysaccharides and gums; polyacrylic acids and the salts thereof; polymethacrylic acids and the salts thereof, methacrylate copolymers; polyvinylalcohol; polyvinylpyrrolidone, copolymers of polyvinylpyrrolidone with vinyl acetate; combinations of polyvinylalcohol and polyvinylpyrrolidone; polyalkylene oxides; and combinations thereof. For example, the hydrophilic rate controlling agent is hydroxypropyl methylcellulose.

The pH-dependent polymer may be one or more of cellulose acetate phthalate; cellulose acetate trimelliate; hydroxypropyl methylcellulose phthalate; hydroxypropyl methylcellulose acetate succinate; polyvinyl acetate phthalate; acrylic acid polymers and copolymers; polymers and copolymers of methacrylic acid and methacrylates, and the like.

The permeation enhancer may be one or more of pore-forming agents, water-soluble compounds and hydrophilic polymers, and the like. For example, the permeation enhancer is polyvinylpyrrolidone, lactose or hydroxypropyl methylcellulose.

The core includes lamotrigine in an amount of at least about 80% (w/w) of the total amount of lamotrigine in the formulation. The amount of hydrophilic rate controlling agent is from about 5% to about 80% (w/w) of the total weight of the formulation. The amount of pH-dependent polymer is from about 1% to about 25% (w/w) of the total weight of the formulation.

The topcoat further of the controlled release formulation may include one or more of hydrophilic polymers, disintegrants and superdisintegrants, diluents, and binders.

### Detailed Description of the Invention

The term "controlled release formulation" as referred to herein is defined to mean oral pharmaceutical formulations which when administered releases the active medicament at a relatively constant rate and provides plasma concentrations of the active medicament that remain substantially invariant with time within the therapeutic range of the active medicament over a 24-hour period and encompasses "prolonged release", "extended release", modified release", "delayed release" and "sustained release" formulations. The formulations as described herein deliver a therapeutically effective amount of lamotrigine to a patient for at least up to 20 hours following a once-daily administration. The term "therapeutically effective amount" is an amount of the active agent which stops or reduces the progress of the condition to be treated or which otherwise completely or partly cures or acts palliatively on the condition. Lamotrigine or a pharmaceutically acceptable salt or derivative thereof may be present from an amount of about 1 mg to about 500 mg in the controlled release formulation. Particularly, the controlled release formulation may comprise lamotrigine or a pharmaceutically acceptable salt or derivative thereof in an amount of 25mg, 50mg, 100mg, 150mg, 200mg, 250mg, and/or 300mg. The recommended dose of Lamictal® XR may also be considered as a standard dose.

The controlled release formulation includes a coating, such that the coating does not include any orifice. The term "orifice" as described herein refers to holes, slits, outlets, cracks, apertures, gaps or channels formed mechanically or *in situ* in the coating which assists in the release of drug from the core.

The "hydrophilic rate controlling agent" as disclosed herein includes without limitation those agents that swell and/or gel in aqueous media. Hydrophilic rate controlling agents suitable for use in the core include alkylcelluloses such as methylcellulose; hydroxyalkylcelluloses, for example, hydroxymethyl cellulose, hydroxyethyl cellulose, hydroxypropyl cellulose and hydroxybutylcellulose; hydroxyalkyl alkylcelluloses such as hydroxyethyl methylcellulose and hydroxypropyl methylcellulose; carboxyalkylcelluloses such as carboxymethylcellulose; alkali metal salts of carboxyalkylcelluloses such as sodium carboxymethylcellulose; carboxyalkyl alkylcelluloses such as carboxymethyl ethylcellulose; carboxyalkylcellulose esters; other natural, semi-synthetic, or synthetic polysaccharides such as alginic acid, alkali metal and ammonium salts thereof, carrageenans, galactomannans, tragacanth, agar-agar, gum arabic, guar gum, xanthan gum, starches, pectins, such as sodium carboxymethyl amylopectin, chitin derivates such as chitosan, polyfructans, inulin; polyacrylic acids and the salts thereof; polymethacrylic acids and the salts thereof, methacrylate copolymers; polyvinylalcohol; polyvinylpyrrolidone, copolymers of polyvinylpyrrolidone with vinyl acetate; combinations of polyvinylalcohol and polyvinylpyrrolidone; polyalkylene oxides such as polyethylene oxide and polypropylene oxide and copolymers of ethylene oxide and propylene oxide; and combinations thereof. In one embodiment, the hydrophilic rate controlling agent(s) are the alkyl celluloses, like hydroxypropyl methylcellulose. Suitable types are sold under the trade name of Methocel by The Dow Chemical Company, such as Methocel® E4M Premium CR, Methocel® K100LV Premium CR, Methocel® K4M, Methocel® K15M, and combinations thereof.

The amount of hydrophilic rate controlling agent may vary from about 1% (w/w) to about 90% (w/w) of the core. The amount of hydrophilic rate controlling agent in the core may vary from about 5% to about 80% by weight of the total weight of the formulation, in particular from about 5% to about 70%, preferrably from about 5% to about 50% by weight of the total weight of the formulation.

The core includes lamotrigine in an amount of at least about 80% by weight of the total amount of lamotrigine in the controlled release formulation as described herein.

The core of the controlled release formulation as described herein may further include one or more of other pharmaceutically acceptable excipient(s). The term "pharmaceutically acceptable excipient(s)" as recited herein includes conventional pharmaceutical additives known in the art, such as diluent(s), binder(s), lubricants(s), granulating solvent(s), glidants(s) or combinations thereof.

Suitable diluents include saccharides like lactose, dextrose, sucrose, fructose, maltose; sugars like mannitol, erythritol, sorbitol, xylitol and lactitol; cellulose derivatives like powdered cellulose, microcrystalline cellulose; dicalcium phosphate, tribasic calcium phosphate, calcium sulphate, calcium carbonate, kaolin and the like.

Suitable binders include starch derivatives like corn starch and pregelatinized starch; cellulose ethers such as carboxymethyl cellulose, methylcellulose, hydroxypropyl cellulose, hydroxypropyl methylcellulose; carboxy vinyl polymers like carbomers; acrylates such as Eudragits; polyvinylpyrrolidone, polyvinylpyrrolidone/vinyl acetate copolymer; xanthan gum, guar gum and other such materials routinely used in the art of solid dosage form manufacturing.

Suitable lubricants include magnesium stearate, calcium stearate, zinc stearate, sodium stearyl fumarate, powdered stearic acid, magnesium oleate, calcium palmitate, potassium laureate, sodium suberate, vegetable oil, mineral oil and the like. Glidants may be selected from talc, colloidal silicon dioxide, corn starch and the like.

Suitable granulating solvents include water, ethanol, methanol, isopropyl alcohol, methylene chloride, acetone, and solutions of binder in these solvents.

The core may further include pharmaceutically acceptable acid or combinations thereof, wherein such an acid may be selected from adipic acid, ascorbic acid, isoascorbic acid, fumaric acid, citric acid, malic acid, succinic acid, tartaric acid, and the like.

Suitable "pH-dependent polymers" include cellulose acetate phthalate; cellulose acetate trimelliate; hydroxypropyl methylcellulose phthalate; hydroxypropyl methylcellulose acetate succinate; polyvinyl acetate phthalate; acrylic acid polymers and copolymers; polymers and copolymers of methacrylic acid and methacrylates (e.g. those available under the trade name Eudragit® from Evonik-Degussa). One or more of such pH-dependent polymers may be incorporated in the said coating. In one embodiment, methacrylate based polymers (e.g. Eudragit® L100-55 and Eudragit® L30D-55) are used. The amount of pH-dependent polymer in the formulation may be present in an amount of from about 1% (w/w) to about 25% (w/w) of the total weight of the controlled release formulation. The pH-dependent polymer may be present in an amount of from about 1% (w/w) to about 95% (w/w) of the coating composition.

The coating composition as described herein may further include one or more of permeation enhancers, plasticizers, coloring agents, anti-tacking agents, lubricants/glidants, solvents and other conventionally used coating additives.

Suitable permeation enhancers includes pore-forming agent(s), water-soluble compounds and hydrophilic polymers, and may be selected from alkali metal salts, e.g. sodium chloride, sodium bromide and the like; alkaline earth metals, e.g. calcium phosphate, calcium nitrate and the like; transition metal salts, e.g. ferric chloride, ferrous sulfate and the like; polyglycols; ethyl vinyl alcohols; glycerin; pentaerythritol; polyvinyl alcohols; vinylpyrrolidone; N-methyl pyrrolidone; saccharides; hydrolyzed starch; pregelatinized starch; carbohydrates, for e.g. glyceraldehydes, erythrose, ribose, arabinose, xylose, glucose, mannose, galactose, maltose, lactose, sucrose and the like; and sugar alcohols, e.g. mannitol and the like. Hydrophilic polymer(s) that may be used as a permeation enhancer may include hydroxypropyl cellulose, hydroxypropyl methylcellulose, polyvinylpyrrolidone and the like.

Suitable plasticizers include dibutyl sebacate, polyethylene glycol, triethyl citrate, triacetin, acetylated triacetin, tributyl citrate, glycerol tributyrate, natural, semi-synthetic and synthetic glycerides, monoglyceride, acetylated monoglycerides, fractionated coconut oil, rape oil, olive oil, sesame oil, castor oil, hydrogenated castor oil, acetyltributylcitrate, acetyltriethylcitrate, glycerin sorbitol, diethyl oxalate, diethyl phthalate, dibutyl phthalate, diethyl malate, diethyl fumarate, dibutyl succinate, diethyl malonate, dioctyl phthalate, and the like.

Suitable anti-tacking agents include adipic acid, magnesium stearate, calcium stearate, zinc stearate, hydrogenated vegetable oils, Sterotex, glyceryl monostearate, talc, silica, sodium benzoate, sodium lauryl sulfate, magnesium lauryl sulfate, and the like.

The coating composition may further include one or more solvents. Suitable solvents for the coating composition may include isopropyl alcohol (IPA), water, methylene chloride and mixtures thereof. Various combinations of solvents may be used such as isopropyl alcohol and water, isopropyl alcohol and methylene chloride and the like.

The controlled release formulation may further be coated with a topcoat of lamotrigine in an immediate release form, wherein the active ingredient is released immediately upon ingestion and thus ensures a rapid onset of action.

The amount of lamotrigine present in the topcoat is at least about 5% by weight of the total amount of lamotrigine present in the controlled release formulation as referred to herein.

The topcoat may further include one or more of hydrophilic polymer(s), disintegrant(s), superdisintegrant(s), diluent(s), binder(s) and other conventionally used pharmaceutically acceptable excipients as described herein.

Suitable disintegrants and superdisintegrants include alginic acid, carboxymethylcellulose calcium, carboxymethylcellulose sodium, colloidal silicon dioxide, croscarmellose sodium, polyvinylpyrrolidone, crosslinked polyvinylpyrrolidone, guar gum, magnesium aluminium silicate, sodium starch glycolate, , corn starch, potato starch, pregelatinized starch, low-substituted hydroxypropylcellulose, methylcellulose, microcrystalline cellulose, polacrilin potassium, powdered cellulose, pregelatinized starch, sodium alginate, and methacrylic acid divinylbenzene copolymer salts.

The controlled release formulation may further include a non-functional coating. The non-functional coating may include any pharmaceutically acceptable colourants or opacifiers including water soluble dyes, aluminium lakes of water soluble dyes and inorganic pigments such as titanium dioxide and iron oxide. It may also contain one or more plasticizing agents conventionally used in polymeric film coatings, for examples, polyethylene glycol, propylene glycol, dibutyl sebacate, mineral oil, sesame oil, diethyl phthalate and triacetin. Proprietary non-functional coating materials, such as Opaspray® and Opadry®, obtainable from Colorcon Ltd., UK, may be used.

The controlled release formulation may also include additional active ingredients, for example, other antiepileptic drugs selected from phenytoin, phosphenytoin, trimethadione, carbamazepine, oxcarbazepine, valproates (valproic acid, sodium valproate, and divalproex sodium), tiagabine, levetiracetam, brivacetam, seletracetam, pregabalin, gabapentin, topiramate, beclamide, felbamate, zonisamide, valpromide, mesuximide, pheneturide and the like.

The "core" of the controlled release formulation as used herein, may be in the form of tablets, minitablets, capsules, granules, pellets, beads or pills. The cores may be formulated following any conventional techniques known in the art, namely dry granulation, aqueous or non-aqueous wet granulation, melt granulation, direct compression, roller compactation, pelletization, melting, intimate and non-intimate blending, kneading, and the like.

The core as described herein may be coated with the respective coating composition(s) using various techniques, such as perforated pan, fluidized bed technique, compression coating, drug layering or spraying.

The controlled release formulations may be compressed into tablets. Alternately, one or more of the controlled release formulation as discussed herein may be dispensed as a unit dosage form. The formulations may be filled in hard-gelatin capsules such that a therapeutically effective amount of the active ingredient is available per dosage form. Alternately, one or more of the controlled release formulations as referred to herein may be packed into an appropriate packaging for single or unit dose administration.

In one embodiment, a controlled release formulation includes
a) a core including lamotrigine, hydrophilic rate controlling agent(s), diluent(s), binder(s), glidant(s) and lubricant(s); and
b) a coating including pH-dependent polymer(s), plasticizer(s), and anti-tacking agent(s);
wherein the said coating does not include any orifice.
In another embodiment, a controlled release formulation includes
a) a core including lamotrigine, hydrophilic rate controlling agent(s), diluent(s), binder(s), glidant(s) and lubricant(s); and
b) a coating including pH-dependent polymer(s), permeation enhancer(s), plasticizer(s), and anti-tacking agent(s).
In yet another embodiment, a controlled release formulation includes
a) a core including lamotrigine, hydrophilic rate controlling agent(s), diluent(s), binder(s), glidant(s) and lubricant(s);
a) a topcoat including lamotrigine in an immediate release form.

In another embodiment, a controlled release formulation includes
a) a core including lamotrigine, hydrophilic rate controlling agent(s), diluent(s), binder(s), glidant(s) and lubricant(s);
b) a coating including pH-dependent polymer(s), permeation enhancer(s), plasticizer(s), and anti-tacking agent(s); and
c) a topcoat including lamotrigine in an immediate release form.

In one aspect of the above embodiments, the hydrophilic rate-controlling agent(s) is hydroxypropyl methylcellulose, polyethylene oxide, xanthan gum, or combinations thereof.

In another aspect of the above embodiments, the pH-dependent polymer(s) is methacrylate copolymer, hydroxypropyl methylcellulose phthalate, or combinations thereof.

In another aspect of the above embodiments, the permeation enhancer(s) is hydroxypropyl methylcellulose, polyvinylpyrrolidone, or combinations thereof.

The invention and background information useful for carrying it out are illustrated by the following examples wherein Examples 3 and 7 illustrate the invention as claimed and Examples 1, 2, 4 to 6 and 8 to 13 represent background information. The invention as described herein may be illustrated by the following examples 3 and 7 but is not to be construed to be limiting by them.

### Examples 1 - 4:

| **Ingredients** | **Quantity (mg)** | | | |
|---|---|---|---|---|
| | **Example 1** | **Example 2** | **Example 3** | **Example 4** |
| **Core** | | | | |
| Lamotrigine | 200.00 | 200.00 | 160.00 | 200.00 |
| Hydroxypropyl methylcellulose (E4M) | 70.00 | 50.00 | 50.00 | 70.00 |
| Hydroxypropyl methylcellulose (K100LV) | - | 50.00 | 50.00 | - |
| Lactose | 80.00 | 80.00 | 80.00 | 80.00 |
| Microcrystalline cellulose | 50.00 | 50.00 | 50.00 | 50.00 |
| Polyvinylpyrrolidone | - | - | - | 12.00 |
| Talc | 3.00 | 3.00 | 3.00 | 3.00 |
| Magnesium stearate | 3.00 | 3.00 | 3.00 | 3.00 |
| Isopropyl alcohol | - | - | - | q.s. |
| **Coating** | | | | |
| Eudragit L30D-55 | 31.23 | 46.72 | 45.69 | 32.15 |
| Hydroxypropyl methylcellulose | 3.12 | - | - | 3.22 |
| Lactose | - | 9.34 | - | - |
| Polyvinylpyrrolidone | - | - | 4.57 | - |
| Triethyl citrate | 3.12 | 4.67 | 4.57 | 3.22 |
| Talc | 3.12 | 4.67 | 4.57 | 3.21 |
| Water | q.s. | q.s. | q.s. | q.s. |
| **Topcoating** | | | | |
| Lamotrigine | - | - | 40.00 | - |
| Hydroxypropyl methylcellulose | - | - | 4.00 | - |
| Crospovidone | - | - | 4.00 | - |
| Isopropyl alcohol: Dichloromethane | - | - | q.s. | - |
| **Total weight** | **446.59** | **501.40** | **503.40** | **459.80** |

### Examples 5 - 8:

| **Ingredients** | **Quantity (mg)** | | | |
|---|---|---|---|---|
| | **Example 5** | **Example 6** | **Example 7** | **Example 8** |
| **Core** | | | | |
| | | | | |
| Lamotrigine | 200.00 | 200.00 | 150.00 | 200.00 |
| Hydroxypropyl | 70.00 | 30.00 | 40.00 | 70.00 |
| methylcellulose | | | | |
| (E4M) | | | | |
| Hydroxypropyl | - | 50.00 | 60.00 | - |
| methylcellulose | | | | |
| (K100LV) | | | | |
| Lactose | 80.00 | 80.00 | 70.00 | 80.00 |
| Microcrystalline | 50.00 | 60.00 | 60.00 | 50.00 |
| cellulose | | | | |
| Polyvinylpyrrolidone | - | - | - | 12.00 |
| Talc | 3.00 | 4.00 | 4.00 | 3.00 |
| Magnesium stearate | 3.00 | 4.00 | 4.00 | 3.00 |
| Isopropyl alcohol | - | - | - | q.s. |
| | | | | |
| **Coating** | | | | |
| | | | | |
| Eudragit L30D-55 | 33.84 | 46.36 | 32.33 | 34.83 |
| Hydroxypropyl | - | - | - | - |
| methylcellulose | | | | |
| Lactose | - | - | - | - |
| Polyvinylpyrrolidone | - | - | - | - |
| Triethyl citrate | 3.38 | 4.64 | 3.23 | 3.48 |
| Talc | 3.38 | 4.64 | 3.23 | 3.48 |
| Water | q.s. | q.s. | q.s. | q.s. |
| | | | | |
| **Topcoating** | | | | |
| | | | | |
| Lamotrigine | - | - | 50.00 | - |
| Hydroxypropyl | - | - | 5.00 | - |
| methylcellulose | | | | |
| Crospovidone | - | - | 5.00 | - |
| Isopropyl alcohol: | - | - | q.s. | - |
| Dichloromethane | | | | |
| | | | | |
| **Total weight** | **446.60** | **483.64** | **486.79** | **459.79** |

### General Procedure for preparation of the tablets:

### Examples 1 and 2:

### Preparation of the Core:

Lamotrigine is blended with the hydrophilic rate controlling agent(s) (hydroxypropyl methylcellulose) and all other excipients that are present in the core (other than magnesium stearate and talc) in a suitable blender and are sifted through sieves of suitable size. The blend is further blended with magnesium stearate and talc. The final blend is compressed into tablets.

### Coating Procedure:

The compressed tablets are loaded in a coating pan and coated using an aqueous dispersion of the pH-dependent polymer (Eudragit® L30D55), permeation enhancer (hydroxypropyl methylcellulose or lactose), plasticizer (triethyl citrate) and anti-tacking agent (talc) until the desired weight gain is achieved.

### Example 3:

### Preparation of the Core:

Eighty percent of the total amount of lamotrigine is blended with the hydrophilic rate controlling agent(s) (hydroxypropyl methylcellulose) and all other excipients that are present in the core (other than magnesium stearate and talc) in a suitable blender and are sifted through sieves of suitable size. The blend is further blended with magnesium stearate and talc. The final blend is compressed into tablets.

### Coating Procedure:

The compressed tablets are loaded in a coating pan and are coated using an aqueous dispersion of the pH-dependent polymer (Eudragit L30D55), permeation enhancer (polyvinylpyrrolidone), plasticizer (triethyl citrate) and anti-tacking agent (talc) until the desired weight gain is achieved.

### Topcoating the coated tablets:

The coated tablets are loaded in a coating pan and are coated using a non-aqueous solution comprising the remaining twenty percent of the total amount of lamotrigine, and the other excipients present in the topcoat.

### Example 4:

### Preparation of the Core:

Lamotrigine is blended with hydrophilic rate controlling agent (hydroxypropyl methylcellulose), microcrystalline cellulose, lactose, and granulated using a solution of polyvinylpyrrolidone in isopropyl alcohol in a rapid mixer granulator. The granules so obtained are dried and subjected to sizing. The sized granules are blended with magnesium stearate and talc. The final blend is compressed into tablets.

### Coating Procedure:

The compressed tablets are loaded in a coating pan and are coated using an aqueous dispersion of the pH-dependent polymer (Eudragit® L30D55), permeation enhancer (hydroxypropyl methylcellulose), plasticizer (triethyl citrate) and anti-tacking agent (talc) until the desired weight gain is achieved.

### Examples 5 and 6:

### Preparation of the Core:

Lamotrigine is blended with the hydrophilic rate controlling agent(s) (hydroxypropyl methylcellulose) and all other excipients that are present in the core (other than magnesium stearate and talc) in a suitable blender and are sifted through sieves of suitable size. The blend is further blended with magnesium stearate and talc. The final blend is compressed into tablets.

### Coating Procedure:

The compressed tablets are loaded in a coating pan and coated using an aqueous dispersion of the pH-dependent polymer (Eudragit® L30D55), plasticizer (triethyl citrate) and anti-tacking agent (talc) until the desired weight gain is achieved.

### Example 7:

### Preparation of the Core:

Seventy-five percent of the total amount of lamotrigine is blended with the hydrophilic rate controlling agent(s) (hydroxypropyl methylcellulose) and all other excipients that are present in the core (other than magnesium stearate and talc) in a suitable blender and are sifted through sieves of suitable size. The blend is further blended with magnesium stearate and talc. The final blend is compressed into tablets.

### Coating Procedure:

The compressed tablets are loaded in a coating pan and are coated using an aqueous dispersion of the pH-dependent polymer (Eudragit L30D55), plasticizer (triethyl citrate) and anti-tacking agent (talc) until the desired weight gain is achieved.

### Topcoating the coated tablets:

The coated tablets are loaded in a coating pan and are coated using a non-aqueous solution comprising the remaining twenty five percent of the total amount of lamotrigine, and the other excipients present in the topcoat.

### Example 8:

### Preparation of the Core:

Lamotrigine is blended with hydrophilic rate controlling agent (hydroxypropyl methylcellulose), microcrystalline cellulose, lactose, and granulated using a solution of polyvinylpyrrolidone in isopropyl alcohol in a rapid mixer granulator. The granules obtained are dried and subjected to sizing. The sized granules are blended with magnesium stearate and talc. The final blend is compressed into tablets.

### Coating Procedure:

The compressed tablets are loaded in a coating pan and are coated using an aqueous dispersion of the pH-dependent polymer (Eudragit L30D55), plasticizer (triethyl citrate) and anti-tacking agent (talc) until the desired weight gain is achieved.

### Examples 9-13

| **Ingredients** | **Quantity (mg)** | | | | |
|---|---|---|---|---|---|
| | **Example 9** | **Example 10** | **Example 11** | **Example 12** | **Example 13** |
| **Core Composition** | | | | | |
| **(Intragranular)** | | | | | |
| | | | | | |
| Lamotrigine | 50.26 | 50.26 | 50.22 | 50.22 | 50.22 |
| Hydroxypropyl | - | - | 50.00 | - | - |
| methylcellulose (K100LV) | | | | | |
| Hydroxypropyl | - | - | 50.00 | - | - |
| methylcellulose (K4M) | | | | | |
| Hydroxypropyl | 55.00 | 60.00 | - | 50.00 | 50.00 |
| methylcellulose (K15M) | | | | | |
| Lactose monohydrate | 79.74 | 79.74 | 110.00 | 79.78 | 79.78 |
| Lactose (Pharmatose | 80.00 | 80.00 | - | 80.00 | 80.00 |
| DCL21) | | | | | |
| Polyvinylpyrrolidone | 15.00 | 15.00 | 20.00 | 17.00 | 15.00 |
| (K30) | | | | | |
| Isopropyl alcohol (IPA) | q.s. | q.s. | q.s. | q.s. | q.s. |
| Purified water | q.s. | q.s. | q.s. | q.s. | q.s. |
| | | | | | |
| **Core Composition** | | | | | |
| **(Extragranular)** | | | | | |
| | | | | | |
| Talc | 3.00 | 3.00 | 3.00 | 3.00 | 3.00 |
| Magnesium stearate | 3.00 | 3.00 | 3.00 | 3.00 | 3.00 |
| | | | | | |
| **Coating Composition** | | | | | |
| | | | | | |
| Eudragit L30D-55 | 12.37 | 14.11 | 12.90 | 12.01 | 11.71 |
| Polyvinylpyrrolidone K25 | 6.18 | 4.23 | - | 4.80 | 5.85 |
| Polyvinylpyrrolidone K30 | - | - | 2.58 | - | - |
| Triethyl citrate | 3.09 | 3.53 | 1.29 | 1.80 | 2.34 |
| Talc | 1.24 | 1.41 | 1.29 | 1.20 | 1.17 |
| Purified Water | q.s. | q.s. | q.s. | q.s. | q.s. |
| | | | | | |
| **Total Weight** | 308.88 | 314.28 | 304.28 | 302.81 | 302.07 |

### General procedure for the preparation of formulations as per Examples 9-13:

### Preparation of Core:

1) Lamotrigine and hydroxypropyl methylcellulose were passed through #22 BSS, followed by lactose, and the sifted material was transferred into a rapid mixer granulator and mixed for 15 minutes using suitable process parameters.
2) Polyvinylpyrrolidone was dissolved in a mixture of IPA: Water under continuous stirring until a clear binder solution was formed.
3) Step-1 premix was granulated with step-2 binder solution using suitable process parameters.
4) Step-3 wet mass was dried in fluid bed drier until the desired moisture in the granules was obtained.
5) Step-4 dry granules were sifted through a suitable sieve and retained granules were milled. Milled and unmilled granules were mixed in a suitable blender.
6) To the step-5 blend, lubricants were added and blended for 5 minutes. The lubricated blend was compressed using suitable punch tooling.

### Preparation of Coating solution & Coating of tablets:

7) Talc was dispersed in purified water under high shear stirring for 10 minutes, to this polyvinylpyrrolidone was added and dissolved under continuous stirring. Triethyl citrate was added and mixed for 15 minutes.
8) Eudragit dispersion was added to the step-7 dispersion at slow speed and mixed for about 30 minutes.
9) Step-6 tablets were transferred into coating pan and coated using the step-8 coating dispersion using suitable process parameters.

Tablets of Examples 9-13 were subjected to *in vitro* dissolution studies in a USP type II apparatus with alternate sinkers, at 75rpm, at a temperature of 37°C±0.5°C in 900mL of 0.1 N hydrochloric acid for the first 2 hours and followed by media replacement with pH 6.8 Phosphate buffer medium. Aliquot of samples were withdrawn at predetermined time intervals and replaced with an equal amount of fresh media. Samples were processed and analysed. Dissolution profiles of these tablets are provided in Table 1.

**Table 1: In vitro release pattern of lamotrigine from controlled release tablets prepared as per composition of Examples 9-13 in USP II apparatus with alternate sinkers, at 75rpm, at a temperature of 37°C±0.5°C in 900mL of 0.1 N hydrochloric acid for the first 2 hours and followed by media replacement with pH 6.8 Phosphate buffer medium.**

| **Time** **(hr)** | **Percent of lamotrigine released from the formulations prepared as per** | | | | |
|---|---|---|---|---|---|
| | **Example 9** | **Example 10** | **Example 11** | **Example 12** | **Example 13** |
| 0 | 0 | 0 | 0 | 0 | 0 |
| 1 | 0 | 0 | 0 | 3 | 0 |
| 2 | 0 | 0 | 0 | 7 | 0 |
| 4 | 9 | 9 | 10 | 28 | 9 |
| 6 | 19 | 18 | 19 | 36 | 20 |
| 8 | 29 | 27 | 28 | 44 | 31 |
| 12 | 48 | 44 | 50 | 61 | 49 |
| 16 | 65 | 58 | 68 | 77 | 64 |
| 20 | 77 | 70 | 86 | 88 | 75 |
| 24 | 85 | 79 | 94 | 97 | 82 |

## Claims

1. A controlled release formulation comprising:
a) a core comprising of lamotrigine and a hydrophilic rate controlling agent;
b) a coating comprising of a pH-dependent polymer and a permeation enhancer; and
c) a topcoat comprising of lamotrigine in immediate release form,
wherein said coating does not include any orifice.

2. The controlled release formulation according to claim 1, wherein the formulation delivers a therapeutically effective amount of lamotrigine for at least up to 20 hours following a once-daily administration.

3. The controlled release formulation according to claim 1, wherein the formulation exhibits the following *in vitro* dissolution profile when measured in a USP type II apparatus with alternate sinkers, at 75 rpm, at a temperature of 37°C±0.5°C in 900mL of 0.1 N hydrochloric acid for the first 2 hours followed by media replacement with pH 6.8 phosphate buffer medium:
a) not more than about 15 % drug released in 2 hours;
b) not more than about 40 % drug released in 4 hours; and
c) not more than about 60 % drug released in 8 hours.

4. The controlled release formulation according to claim 1, wherein the formulation is a unit dosage form.

5. The controlled release formulation according to claim 1, wherein the hydrophilic rate controlling agent comprises alkylcelluloses; hydroxyalkyl alkylcelluloses; carboxyalkylcelluloses; alkali metal salts of carboxyalkylcelluloses; carboxyalkyl alkylcelluloses; carboxyalkylcellulose esters; other natural, semi-synthetic, or synthetic polysaccharides and gums; polyacrylic acids and the salts thereof; polymethacrylic acids and the salts thereof, methacrylate copolymers; polyvinylalcohol; polyvinylpyrrolidone, copolymers of polyvinylpyrrolidone with vinyl acetate; combinations of polyvinylalcohol and polyvinylpyrrolidone; polyalkylene oxides; and combinations thereof.

6. The controlled release formulation according to claim 5, wherein the hydrophilic rate controlling agent comprises hydroxypropyl methylcellulose.

7. The controlled release formulation according to claim 1, wherein the pH-dependent polymer comprises cellulose acetate phthalate; cellulose acetate trimelliate; hydroxypropyl methylcellulose phthalate; hydroxypropyl methylcellulose acetate succinate; polyvinyl acetate phthalate; acrylic acid polymers and copolymers; polymers and copolymers of methacrylic acid and methacrylates.

8. The controlled release formulation according to claim 1, wherein the permeation enhancer comprises pore-forming agents, water-soluble compounds and hydrophilic polymers.

9. The controlled release formulation according to claim 8, wherein the permeation enhancer comprises polyvinylpyrrolidone, lactose or hydroxypropyl methylcellulose.

10. The controlled release formulation according to claim 1, wherein the core comprises lamotrigine in an amount of at least about 80% (w/w) of the total amount of lamotrigine in the formulation.

11. The controlled release formulation according to claim 1, wherein the amount of hydrophilic rate controlling agent comprises about 5% to about 80% (w/w) of the total weight of the formulation.

12. The controlled release formulation according to claim 1, wherein the amount of pH-dependent polymer comprises about 1% to about 25% (w/w) of the total weight of the formulation.

13. The controlled release formulation according to claim 1, wherein the topcoat further comprises one or more of hydrophilic polymers, disintegrants and superdisintegrants, diluents, and binders.

## Patentansprüche

1. Formulierung mit gesteuerter Freisetzung, umfassend:
a) einen Kern, bestehend aus Lamotrigin und einem die Wasseraufnahmerate steuernden Agens;
b) einen Überzug, bestehend aus einem pH-abhängigen Polymer und einem Permeationsbeschleuniger; und
c) eine Deckschicht aus Lamotrigin in der Form für unmittelbare Freisetzung,
wobei der Überzug keine Öffnung aufweist.

2. Formulierung mit gesteuerter Freisetzung gemäß Anspruch 1, wobei die Formulierung nach einmaliger Verabreichung bis zu zwanzig Stunden eine therapeutisch wirksame Lamotrigin-Menge abgibt.

3. Formulierung mit gesteuerter Freisetzung gemäß Anspruch 1, wobei die Formulierung bei zweistündiger Messung in einer USP-Vorrichtung Typ II mit alternierenden Sinkern bei 75 U/min und einer Temperatur von 37°C ± 0,5°C in 900 ml einer 0,1 N Hydrochlorsäure und nach einem anschließenden Austausch des Mediums gegen ein Phosphatpuffermedium das folgende in vitro-Auflösungsprofil zeigt:
a) eine Freisetzung von nicht mehr als 15% der Arznei in zwei Stunden;
b) eine Freisetzung von nicht mehr als 40% der Arznei in vier Stunden und
c) eine Freisetzung von nic.ht mehr als 60% der Arznei in acht Stunden.

4. Formulierung mit gesteuerter Freisetzung gemäß Anspruch 1, wobei die Formulierung eine Dosierungseinheitsform ist.

5. Formulierung mit gesteuerter Freisetzung gemäß Anspruch 1, wobei das Agens zur Steuerung der Wasseraufnahmerate Alkylcellulosen; Hydroxyalkylalkylcellulosen; Carboxyalkylcellulosen; Alkalimetallsalze von Carboxyalkylcellulosen; Carboxyalkylalkylcellulosen; Carboxyalkylalkylcellulose-Ester; andere natürliche, halbsynthetische oder synthetische Polysaccharide und Gummiharze; Polyacrylsäuren und deren Salze; Polymethacrylsäuren und deren Salze; Methacrylatcopolymere; Polyvinylalkohol; Polyvinylpyrrolidon, Compolymere von Polyvinylpyrrolidon mit Vinylacetat; Kombinationen von Polyvinylalkohol und Polyvinylpyrrolidon; Polyalkylenoxide und Kombinationen davon.

6. Formulierung mit gesteuerter Freisetzung gemäß Anspruch 5, wobei das die die Wasseraufnahmerate steuernde Agens Hydroxypropylmethylcellulose enthält.

7. Formulierung mit gesteuerter Freisetzung gemäß Anspruch 1, wobei das pHabhängige Polymer Celluloseacetatphthalat; Celluloseacetat-trimelliat; Hydroxypropylmethylcellulosephthalat; Hydroxypropylmethylcelluloseacetatsuccinat; Polyvinylacetatphthalat; Acrylsäurepolymere und -copolymere; Polymere und Copolymere von Methylacrylsäure und Methylacrylat.

8. Formulierung mit gesteuerter Freisetzung gemäß Anspruch 1, wobei der Permeationsbeschleuniger Porenbildner, wasserlösliche Verbindungen und hydrophile Polymere enthält.

9. Formulierung mit gesteuerter Freisetzung gemäß Anspruch 8, wobei der Permeationsbeschleuniger Polyvinylpyrrolidon, Lactose oder Hydroxypropylmethylcellulose enthält.

10. Formulierung mit gesteuerter Freisetzung gemäß Anspruch 1, wobei der Kern Lamotrigin in einer Menge von mindestens etwa 80% (w/w) der Gesamtmenge an Lamotrigin in der Formulierung enthält.

11. Formulierung mit gesteuerter Freisetzung gemäß Anspruch 1, wobei die Menge des die Wasseraufnahmerate steuernden Agens etwa 5% bis etwa 8% (w/w) des Gesamtgewichts der Formulierung ausmacht.

12. Formulierung mit gesteuerter Freisetzung gemäß Anspruch 1, wobei die Menge des pH-abhängigen Polymers etwa 1 % bis etwa 25% (w/w) des Gesamtgewichts der Formulierung ausmacht.

13. Formulierung mit gesteuerter Freisetzung gemäß Anspruch 1, wobei die Deckschicht ferner ein oder mehrere hydrophile Polymere, Disintegrationsmittel und Superdisintegrationsmittel, Verdünnungsmittel und Bindemittel enthält.

## Revendications

1. Formulation à libération contrôlée, comprenant :
a) un noyau constitué de lamotrigine et d'un agent de contrôle de débit hydrophile ;
b) un revêtement constitué d'un polymère fonction du pH et d'un promoteur de perméation ; et
c) une couche supérieure constituée de lamotrigine sous forme à libération immédiate,
dans laquelle ledit revêtement ne comprend pas d'orifice quelconque.

2. Formulation à libération contrôlée selon la revendication 1, dans laquelle la formulation délivre une quantité efficace au plan thérapeutique de lamotrigine pendant au moins jusqu'à 24 heures après une administration quotidienne unique.

3. Formulation à libération contrôlée selon la revendication 1, dans laquelle la formulation présente le profil de dissolution *in vitro* suivant lorsqu'il est mesuré dans un appareil USP de type II avec des plombs alternés à 75 tr/min à une température de 37 °C +/- 0,5 °C dans 900 mL d'acide chlorhydrique 0,1N pendant les 2 premières heures, le tout suivi d'un remplacement de milieu par un milieu tampon de phosphate à pH 6,8 :
a) pas plus d'environ 15 % de médicament libérés en 2 heures ;
b) pas plus d'environ 40 % de médicament libérés en 4 heures ; et
c) pas plus d'environ 60 % de médicament libérés en 8 heures.

4. Formulation à libération contrôlée selon la revendication 1, dans laquelle la formulation est une forme posologique unitaire.

5. Formulation à libération contrôlée selon la revendication 1, dans laquelle l'agent de contrôle de débit hydrophile comprend les alkylcelluloses ; les hydroxyalkylalkylcelluloses ; les carboxyalkylcelluloses ; les sels de métaux alcalins de carboxyalkylcelluloses ; les carboxyalkylalkyl-celluloses ; les esters de carboxyalkylcelluloses ; d'autres polysaccharides et gommes naturels, semi-synthétiques ou synthétiques ; les acides polyacryliques et leurs sels ; les acides polyméthacryliques et leurs sels, les copolymères de méthacrylates ; l'alcool polyvinylique ; la polyvinylpyrrolidone, les copolymères de polyvinylpyrrolidone avec de l'acétate de vinyle ; les combinaisons d'alcool polyvinylique et de polyvinylpyrrolidone ; les poly(oxydes d'alkylène) ; et leurs combinaisons.

6. Formulation à libération contrôlée selon la revendication 5, dans laquelle l'agent de contrôle de débit hydrophile comprend l'hydroxypropyl-méthylcellulose.

7. Formulation à libération contrôlée selon la revendication 1, dans laquelle le polymère fonction du pH comprend l'acétate phtalate de cellulose ; l'acétate trimelliate de cellulose ; le phtalate d'hydroxypropylméthylcellulose ; l'acétate succinate d'hydroxypropylméthyl-cellulose ; l'acétate phtalate de polyvinyle ; les polymères et les copolymères d'acide acrylique ; les polymères et copolymères d'acide méthacrylique et les méthacrylates.

8. Formulation à libération contrôlée selon la revendication 1, dans laquelle le promoteur de perméation comprend des agents porogènes, des composés solubles dans l'eau et des polymères hydrophiles.

9. Formulation à libération contrôlée selon la revendication 8, dans laquelle le promoteur de perméation comprend la polyvinylpyrrolidone, le lactose ou l'hydroxypropylméthylcellulose.

10. Formulation à libération contrôlée selon la revendication 1, dans laquelle le noyau comprend de la lamotrigine en quantité d'au moins environ 80 % (en poids/poids) de la quantité totale de lamotrigine dans la formulation.

11. Formulation à libération contrôlée selon la revendication 1, dans laquelle la quantité d'agent de contrôle de débit hydrophile comprend environ 5 % à environ 80 % (en poids/poids) du poids total de la formulation.

12. Formulation à libération contrôlée selon la revendication 1, dans laquelle la quantité de polymère fonction du pH comprend environ 1 % à environ 25 % (en poids/poids) du poids total de la formulation.

13. Formulation à libération contrôlée selon la revendication 1, dans laquelle la couche supérieure comprend en outre un ou plusieurs polymères hydrophiles, désintégrants et superdésintégrants, diluents et liants.
